# EUROPEAN PATENT APPLICATION

(11) **EP 4 840 507 A2**
(43) Date of publication of application: **18.11.2026**
(21) Application number: 26192823.8
(22) Date of filing: 08.01.2020
(51) Int. Cl.: A61P 3/10

(54) **SMALL MOLECULE ANALOGS OF THE PROTEIN E4ORF1 IN THE TREATMENT AND PREVENTION OF METABOLIC DISORDERS**

(30) Priority: 08.01.2019 US 201962789674 P
(62) Document number(s) of the earlier application(s) in accordance with Art. 76 EPC: 20738769.7 / 3 908 308
(71) Applicant: Texas Tech University System, Lubbock TX 79409-2021 (US)
(72) Inventor: Dhurandhar, Nikhil V., Lubbock, 79409-2021 (US); Das, Bhaskar, C., Lubbock, 79409-2021 (US)
(74) Representative: Murgitroyd & Company

(57) **Abstract**

In an embodiment, the present disclosure pertains to compositions and methods for modulating cellular glucose uptake. In general, the methods include associating cells with the compositions of the present disclosure. In another aspect, the present disclosure pertains to compositions and methods to treat or prevent a disorder in a subject. The methods generally include administering the compositions of the present disclosure to the subject.

## Description

### CROSS-REFERENCE TO RELATED APPLICATIONS

This application claims priority to U.S. Provisional Patent Application No. 62/789,674, filed on January 8, 2019. The entirety of the aforementioned application is incorporated herein by reference.

### BACKGROUND

Early 4 open reading frame 1 protein of human adenovirus 36 (E4orf1) has been effective in glycemic control. However, without an effective delivery method, E4orf1 has limited abilities in treating human patients. For example, there is no receptor for E4orf1 in human cells, which makes it challenging to deliver the E4orf1 protein to humans. The present disclosure seeks to circumvent this limitation by using small molecule analogs of E4orf1 to modulate cellular glucose uptake.

### SUMMARY

In some embodiments, the present disclosure pertains to a method of modulating cellular glucose uptake. The method can generally include associating cells with a composition. In some embodiments, the composition can include one or more of the following compounds: 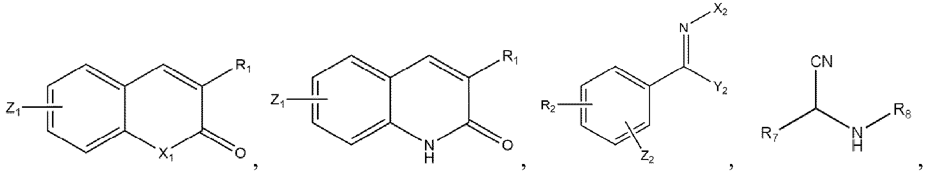 or combinations thereof.

In some embodiments, the composition can include one or more of the following compounds: 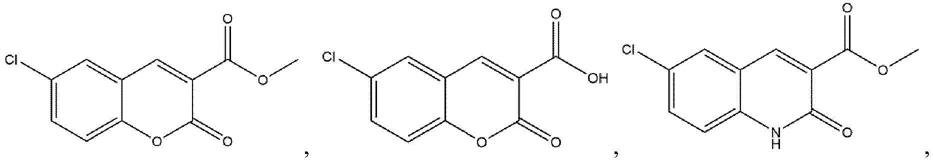 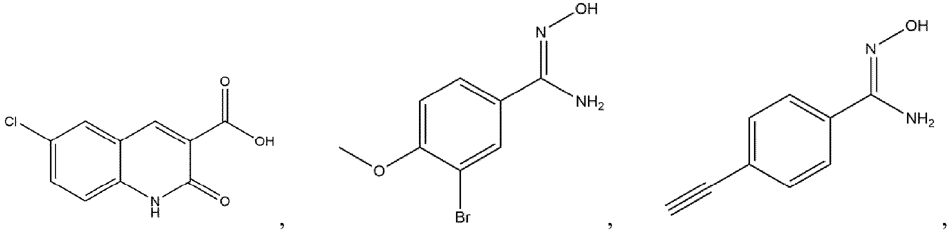 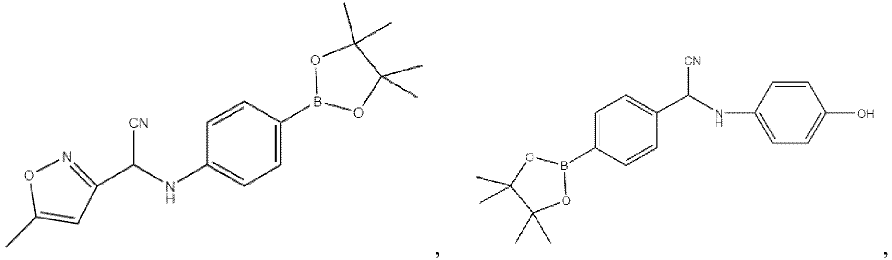 or combinations thereof.

In some embodiments, the association of the compositions of the present disclosure with cells is performed *in vitro* or *in vivo* in a subject. In some embodiments, the associating includes administering one or more of the compositions outlined above to the subject. In some embodiments, the administering is performed orally, intramuscularly, intranasally, subcutaneously, intra- or trans-dermally, intravenously, or through combinations of such methods.

In some embodiments, the aforementioned compositions are used to treat or prevent a disorder in a subject. In some embodiments, the disorder is prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof.

In some embodiments, the aforementioned compositions increase cellular glucose uptake. In some embodiments, the aforementioned compositions increase cellular glucose uptake in fat cells, muscle cells, or liver cells. In some embodiments, the aforementioned compositions increase cellular glucose uptake independently of proximal insulin signaling pathways. In some embodiments, the aforementioned compositions mimic function of early 4 open reading frame 1 protein of human adenovirus 36 (E4orf1). In some embodiments, the aforementioned compositions mimic function of E4orf1 in glucose uptake and insulin signaling in cells. In some embodiments, the aforementioned compositions cause an increase in pAkt/Akt in cells.

In some embodiments, the compositions of the present disclosure can reduce endogenous insulin levels in the blood. In some embodiments, the compositions of the present disclosure improve blood glucose levels by reducing high levels of glucose in the blood, or reducing the rise in glucose levels or the duration of glucose elevation expected after eating food. In some embodiments, the compositions of the present disclosure reduce glucose output from the liver. In some embodiments, the compositions of the present disclosure prevent an increase in blood insulin levels, reduce production and secretion of insulin while improving blood glucose levels, and thereby prevent damage to pancreatic cells that make insulin.

In some embodiments, the compositions of the present disclosure reduce fat accumulation in the liver. In some embodiments, the compositions of the present disclosure prevent accumulation of fat in the liver or reduce accumulated lipid from the liver.

In some embodiments, the compositions of the present disclosure are associated with delivery agents, such as nanoparticles. In some embodiments, the compositions of the present disclosure are associated with one or more solubilizing agents.

In some embodiments, the present disclosure pertains to a method of treating or preventing a disorder in a subject. The method can generally include administering one or more of the compositions of the present disclosure to the subject. In some embodiments, the disorder can include, without limitation, prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof. In some embodiments, the administering can be performed orally, intramuscularly, intranasally, subcutaneously, intra- or trans-dermally, intravenously, or combinations thereof.

### DESCRIPTION OF THE DRAWINGS

**FIGURE 1** illustrates a method of modulating cellular glucose uptake in cells.
**FIGURE 2** depicts the structures of various active E4orf1 analogs.
**FIGURE 3** illustrates that selected E4orf1 chemical analogs increase glucose uptake. 3T3L1 preadipocytes were treated with dimethyl sulfoxide (DMSO) or insulin or treated with analogs BT324, BT325, BT326, BT327, BT328, and BT329. The treatments were compared using one way analysis of variance (ANOVA) and post hoc Tukey's test. Groups sharing same letters are not statistically significant.
**FIGURE 4** illustrates that the E4orf1 chemical analogs that increase glucose uptake also increase pAkt levels - the underlying mechanism for increasing cellular glucose uptake. 3T3L1 preadipocytes were treated with DMSO or insulin or with analogs BT324, BT325, BT327, or BT329. Post 24 hr incubation in the dark, cell lysates were collected and 20 µg of protein was loaded and probed for pAkt/Akt. Band intensities were analyzed using Image J software. Groups sharing same letters are not statistically significant.
**FIGURE 5** illustrates synthesis of lead molecules BN#3 and BT#141.
**FIGURE 6** illustrates screening of small molecules for potential to increase cellular glucose uptake.
**FIGURE 7** illustrates improved glucose disposal and lower insulin secretion in BN#3 treated mice (mean ± standard deviation).
**FIGURE 8** illustrates improved glucose disposal in BN#141 treated mice (mean ± standard deviation).

### DETAILED DESCRIPTION

It is to be understood that both the foregoing general description and the following detailed description are illustrative and explanatory, and are not restrictive of the subject matter, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that include more than one unit unless specifically stated otherwise.

The section headings used herein are for organizational purposes and are not to be construed as limiting the subject matter described. All documents, or portions of documents, cited in this application, including, but not limited to, patents, patent applications, articles, books, and treatises, are hereby expressly incorporated herein by reference in their entirety for any purpose. In the event that one or more of the incorporated literature and similar materials defines a term in a manner that contradicts the definition of that term in this application, this application controls.

According to the National Institutes of Health, more than 1 in 3 adults are considered to have obesity. In addition, 1 in 6 children and adolescents ages 2 to 19 are considered to have obesity. The prevalence of obesity in the developed world is increasing. Becoming obese puts individuals at risk for several different health conditions, including hypertension, dyslipidemia, heart disease, stroke, and diabetes.

Applicants of the present disclosure have spent many years researching the mechanisms of obesity and diabetes. Applicants have discovered a virus that showed a strong correlation with the development of obesity in humans (referred to as infectobesity). The virus is part of the adenoviridae family and is known as human adenovirus 36. This virus was found within the fat cells of obese and diabetic humans and animals. When experimental animals such as mice or rats are infected with this virus, they improve glucose levels and reduce the requirement of endogenous insulin.

Applicants discovered that a protein made by human adenovirus 36, early 4 open reading frame 1 (E4orf1), seemed to decrease fat in the liver (leading to a cleaner liver), increase the uptake of glucose in fat and muscle cells (which lowers blood sugar levels), and lower the levels of endogenous insulin in animal models. These effects can be utilized to treat and prevent various metabolic disorders, including diabetes.

While E4orf1 has a lot of therapeutic potential, there are still challenges with its ability to treat human patients. In particular, there is no receptor for E4orf1 in human cells. As such, a need exists for more effective compositions and methods for maximizing the therapeutic potential of E4orf1. Various embodiments of the present disclosure address the aforementioned need.

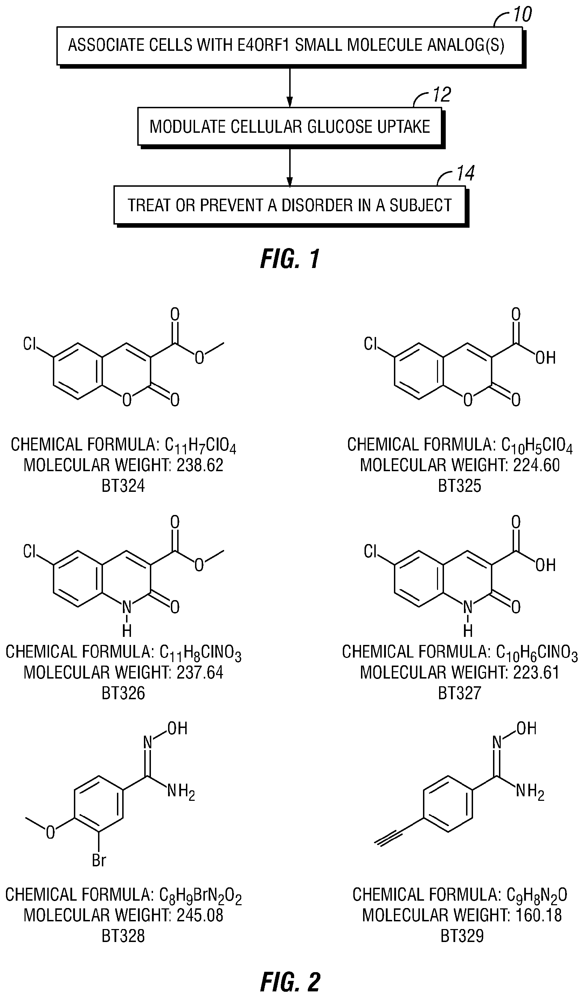

In some embodiments, the present disclosure pertains to methods of modulating cellular glucose uptake. In some embodiments illustrated in **FIG. 1**, the methods of the present disclosure include a step of associating cells with one or more compositions that include one or more E4orf1 small molecule analogs (step 10). The association results in the modulation of cellular glucose uptake (step 12). In some embodiments where the association occurs *in vivo* in a subject, the method can be utilized to treat or prevent a disorder in the subject (step 14).

As set forth in more detail herein, the compositions and methods of the present disclosure can have numerous embodiments. For instance, the compositions of the present disclosure can include various E4orf1 small molecule analogs with various chemical configurations and moieties. Furthermore, various methods may be utilized to modulate cellular glucose uptake in order to treat or prevent various disorders in various subjects.

### Compositions

The compositions of the present disclosure can include various E4orf1 small molecule analogs. For instance, in some embodiments, the compositions of the present disclosure can include: 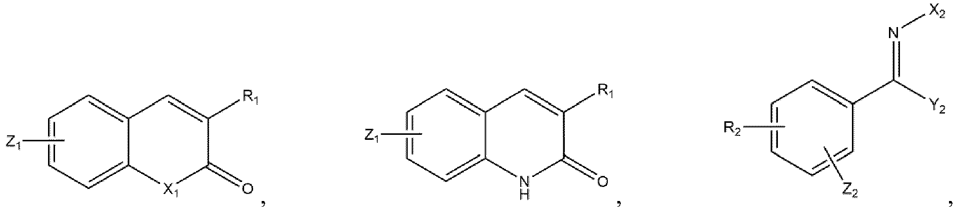 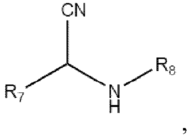 or combinations thereof.

In some embodiments, R₁ can be an alkyl group, an alkene group, an alkyne group, an alkoxyl group, an aldehyde, a carboxyl group, COOR₃, COOH, hydrogen, SH, SH derivatives, SR₆, or OH. In some embodiments, R₃ can be an alkyl group, an alkene group, an alkyne group, a methyl group, an ethyl group, a propyl group, a butyl group, or a pentyl group. In some embodiments, R₆ can be an alkyl group, an alkene group, an alkyne group, an alkoxyl group, an aldehyde, a carboxyl group, COOR₃, COOH, hydrogen, or OH.

In some embodiments, Z₁ and Z₂ each, independently, can be a halogen, hydrogen, SH, SH derivatives, an alkyl group, an alkene group, an alkyne group, or SR₆. In some embodiments, X₁ can be O, NH, NR₄, SH, SH derivatives, CH₂, SR₆, or S. In some embodiments, R₄ can be an alkyl group, an alkene group, an alkyne group, or a hydroxyl group. In some embodiments, R₂ can be OR₅, an alkyl group, an alkene group, an ethynyl group, an alkyne group, SH, SH derivatives, SR₆, or COOR₅. In some embodiments, R₅ can be hydrogen, an alkyl group, an alkene group, an alkyne group, a methyl group, an ethyl group, a propyl group, a butyl group, and a pentyl group. In some embodiments, X₂ and Y₂ each, independently, can be OH, NH₂, NR₄, an alkyl group, an alkene group, an alkyne group, a methyl group, an ethyl group, a propyl group, a butyl group, a pentyl group, SH, SH derivatives, or SR₆.

In some embodiments, R₇ and R₈ can each, independently, be 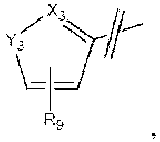 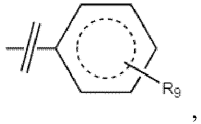 an alkyl group, an alkene group, an alkyne group, an alkoxyl group, an ethynyl group, an aldehyde, a carboxyl group, OH, COOR₃, COOH, hydrogen, SH, SH derivatives, SR₆, OR₅, or COOR₅. In some embodiments, X₃ can be N, CH, SH, SH derivatives, or P. In some embodiments, Y₃ can be O, NH, NR₄, SH, SH derivatives, SR₆, CH₂, or S. In some embodiments, R₉ can be an alkyl group, an alkene group, an alkyne group, an alkoxyl group, an ethynyl group, an aldehyde, a carboxyl group, COOR₃, COOH, hydrogen, SH, SH derivatives, SR₆, OR₅, COOR₅, CH₃, OH, or

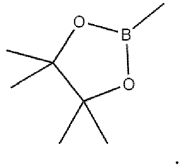

In some embodiments, R₃ can be alkyl group, an alkene group, an alkyne group, a methyl group, an ethyl group, a propyl group, a butyl group, or a pentyl group. In some embodiments, R₅ can be hydrogen, an alkyl group, an alkene group, an alkyne group, a methyl group, an ethyl group, a propyl group, a butyl group, and a pentyl group. In some embodiments, R₆ can be an alkyl group, an alkene group, an alkyne group, an alkoxyl group, an aldehyde, a carboxyl group, COOR₃, COOH, hydrogen, or OH.

In particular embodiments, the compositions of the present disclosure can include: 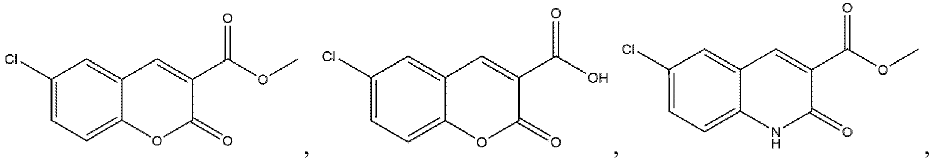 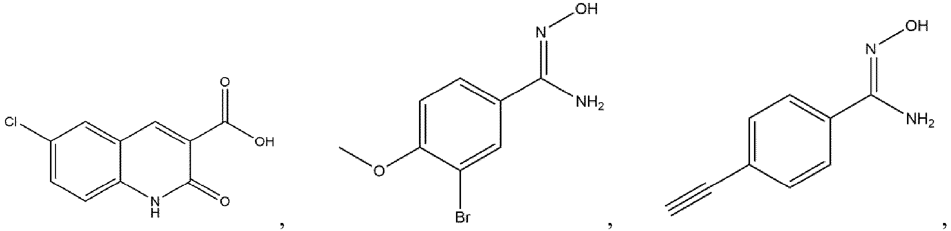 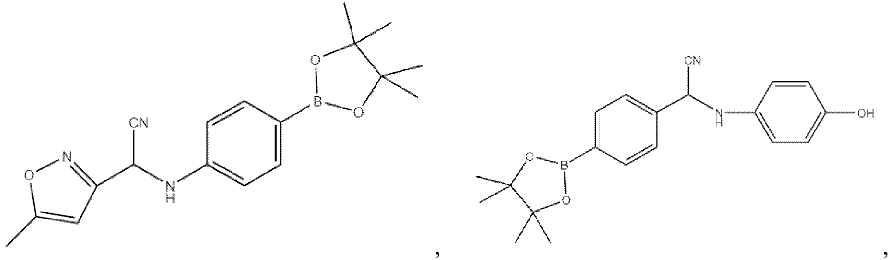 or combinations thereof.

In some embodiments, the compositions of the present disclosure can include Compound 1, as shown herein.

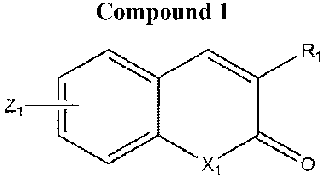

In some embodiments, Compound 1 can include various moieties in various configurations to make up Compound 1, including R₁, X₁, and Z₁. In some embodiments, R₁ can be an alkyl group, an alkene group, an alkyne group, an alkoxyl group, an aldehyde, a carboxyl group, COOR₃, COOH, hydrogen, SH, SH derivatives, SR₆, or OH. In some embodiments, R₃ can be an alkyl group, an alkene group, an alkyne group, a methyl group, an ethyl group, a propyl group, a butyl group, or a pentyl group.

In some embodiments, Z₁ can be a halogen, hydrogen, SH, SH derivatives, an alkyl group, an alkene group, an alkyne group, or SR₆. In some embodiments, X₁ can be O, NH, NR₄, SH, SH derivatives, CH₂, SR₆, or S. In some embodiments, R₄ can be an alkyl group, an alkene group, an alkyne group, or a hydroxyl group.

In some embodiments, R₁, Z₁, and X₁ can be SH, SR₆, SH derivatives, or combinations thereof. In some embodiments, R₆ can be an alkyl group, an alkene group, an alkyne group, an alkoxyl group, an aldehyde, a carboxyl group, COOR₃, COOH, hydrogen, or OH.

In a particular embodiment of Compound 1, R₁ is COOCH₃, X₁ is O, and Z₁ is Cl, as shown herein as compound BT324.

### BT324

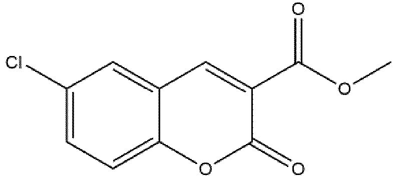

In a particular embodiment of Compound 1, R₁ is COOH, X₁ is O, and Z₁ is Cl, as shown herein as compound BT325.

### BT325

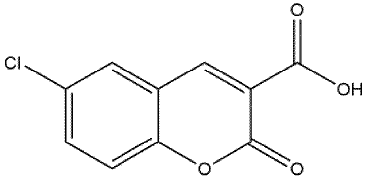

Moreover, in addition to Compound 1, as set forth in more detail herein, the compositions and methods of the present disclosure can include other configurations and/or moieties. For instance, in some embodiments, the compositions of the present disclosure can include a general configuration to that of Compound 2, as shown herein.

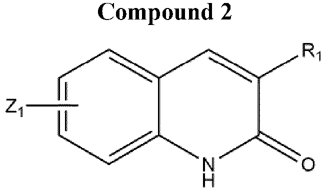

In some embodiments, Compound 2 can include various moieties in various configurations to make up Compound 2, and can include, but are not limited to, R₁ and Z₁.

In some embodiments, R₁ can be an alkyl group, an alkene group, an alkyne group, an alkoxyl group, an aldehyde, a carboxyl group, COOR₃, COOH, hydrogen, SH, SH derivatives, SR₆, or OH. In some embodiments, R₃ can be an alkyl group, an alkene group, an alkyne group, a methyl group, an ethyl group, a propyl group, a butyl group, or a pentyl group. In some embodiments, Z₁ can be a halogen, hydrogen, SH, SH derivatives, an alkyl group, an alkene group, an alkyne group, or SR₆.

In some embodiments, R₁ and Z₁, can be SH, SR₆, SH derivatives, or combinations thereof. In some embodiments, R₆ can be an alkyl group, an alkene group, an alkyne group, an alkoxyl group, an aldehyde, a carboxyl group, COOR₃, COOH, hydrogen, or OH.

In a particular embodiment of Compound 2, R₁ is COOCH₃ and Z₁ is Cl, as shown herein as compound BT326.

### BT326

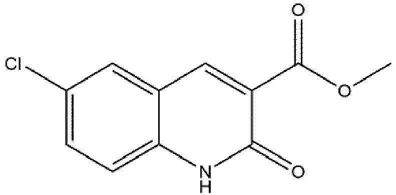

In a particular embodiment of Compound 2, R₁ is COOH and Z₁ is Cl, as shown herein as compound BT327.

### BT327

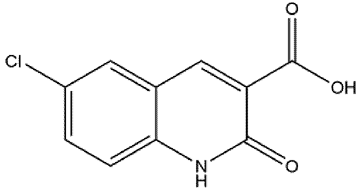

Furthermore, in addition to Compound 1 and Compound 2, as set forth in more detail herein, the compositions and methods of the present disclosure can include various different configurations and/or different moieties. In some embodiments, the compositions of the present disclosure can include Compound 3, as shown herein.

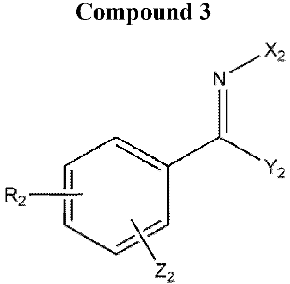

In some embodiments, Compound 3 can include various moieties in various configurations to make up Compound 3, and can include, but are not limited to, R₂, X₂, Y₂, and Z₂. In some embodiments, R₂ can be OR₅, an alkyl group, an alkene group, an ethynyl group, an alkyne group, SH, SH derivatives, SR₆, or COOR₅. In some embodiments, R₅ can be hydrogen, an alkyl group, an alkene group, an alkyne group, a methyl group, an ethyl group, a propyl group, a butyl group, and a pentyl group. In some embodiments, Z₂ can be a halogen, hydrogen, SH, SH derivatives, an alkyl group, an alkene group, an alkyne group, or SR₆.

In some embodiments, X₂ and Y₂ each, independently, can be OH, NH₂, NR₄, an alkyl group, an alkene group, an alkyne group, a methyl group, an ethyl group, a propyl group, a butyl group, a pentyl group, SH, SH derivatives, or SR₆. In some embodiments, R₄ can be an alkyl group, an alkene group, an alkyne group, or a hydroxyl group.

In some embodiments, R₂, X₂, Y₂, and Z₂ and can be SH, SR₆, SH derivatives, or combinations thereof. In some embodiments, R₆ can be an alkyl group, an alkene group, an alkyne group, an alkoxyl group, an aldehyde, a carboxyl group, COOR₃, COOH, hydrogen, or OH.

In a particular embodiment of Compound 3, R₂ is OCH₃, Z₂ is Br, X₂ is OH, and Y₂ is NH₂, as shown herein as compound BT328.

### BT328

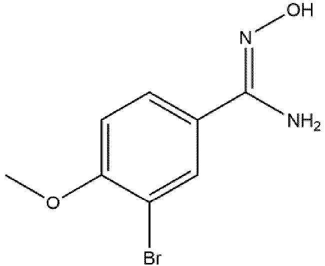

In a particular embodiment of Compound 3, R₂ is ethynyl (CCH), Z₂ is hydrogen, X₂ is OH, and Y₂ is NH₂, as shown herein as compound BT329.

### BT329

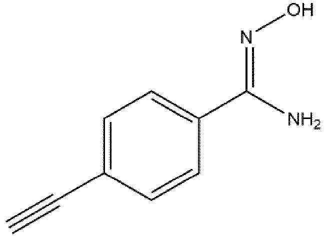

Moreover, in addition to the compounds outlined above, as set forth in more detail herein, the compositions and methods of the present disclosure can include further different configurations and/or different moieties. In some embodiments, the compositions of the present disclosure can include Compound 4, as shown herein.

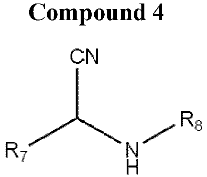

In some embodiments, Compound 4 can include various moieties in various configurations to make up Compound 4, and can include, but are not limited to, R₇ and R₈. In some embodiments, R₇ and R₈ each, independently, can be 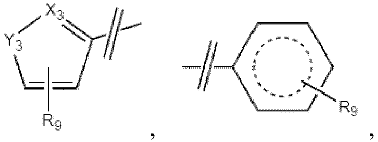 an alkyl group, an alkene group, an alkyne group, an alkoxyl group, an ethynyl group, an aldehyde, a carboxyl group, OH, COOR₃, COOH, hydrogen, SH, SH derivatives, SR₆, OR₅, or COOR₅. In some embodiments, R₃ can be an alkyl group, an alkene group, an alkyne group, a methyl group, an ethyl group, a propyl group, a butyl group, or a pentyl group. In some embodiments, R₅ can be hydrogen, an alkyl group, an alkene group, an alkyne group, a methyl group, an ethyl group, a propyl group, a butyl group, and a pentyl group. In some embodiments, R₆ can be an alkyl group, an alkene group, an alkyne group, an alkoxyl group, an aldehyde, a carboxyl group, COOR₃, COOH, hydrogen, or OH.

In some embodiments, X₃ can be N, CH, SH, SH derivatives, or P. In some embodiments, Y₃ can be O, NH, NR₄, SH, SH derivatives, SR₆, CH₂, or S. In some embodiments, R₄ can be an alkyl group or a hydroxyl group.

In some embodiments, R₉ can be an alkyl group, an alkene group, an alkyne group, an alkoxyl group, an ethynyl group, an aldehyde, a carboxyl group, COOR₃, COOH, hydrogen, SH, SH derivatives, SR₆, OR₅, COOR₅, CH₃, OH, or

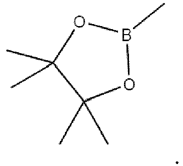

In a particular embodiment of Compound 4, R₇ is 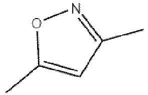 and R₈ is 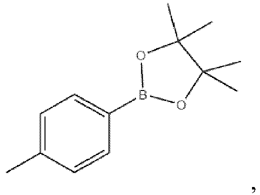 as shown herein as compound BN#3.

### BN#3

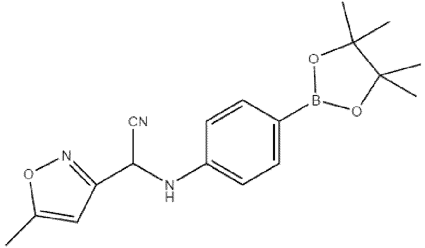

In a particular embodiment of Compound 4, R₇ is 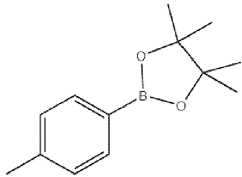 and R₈ is 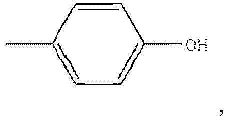 as shown herein as compound BT#141.

### BT#141

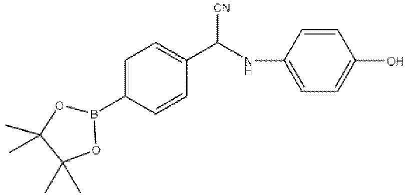

In some embodiments, the compositions of the present disclosure can contain one or more of the aforementioned compounds. In some embodiments, the compositions of the present disclosure can also have one or more physiologically acceptable carriers or excipients.

In some embodiments, the compositions of the present disclosure can also include formulation materials for modifying, maintaining, or preserving various conditions, including pH, osmolarity, viscosity, clarity, color, isotonicity, odor, sterility, stability, rate of dissolution or release, and/or adsorption or penetration of the compounds. Suitable formulation materials include, but are not limited to: amino acids (e.g., glycine); antimicrobials; antioxidants (e.g., ascorbic acid); buffers (e.g., Tris-HCl); bulking agents (e.g., mannitol and glycine); chelating agents (e.g., EDTA); complexing agents (e.g., hydroxypropyl-beta-cyclodextrin); and the like.

### Association of Compositions with Cells

The compositions of the present disclosure may become associated with cells in various manners. For instance, in some embodiments, the compositions of the present disclosure become associated with cells by exposing the cells to the compositions. In some embodiments, the compositions of the present disclosure become associated with cells by incubating the cells with the compositions. In some embodiments, the compositions of the present disclosure become associated with cells by contacting the cells with the compositions.

In some embodiments, the cells can be fat cells, muscle cells, and/or liver cells. In some embodiments, the compositions of the present disclosure become associated with cells *in vitro*. In some embodiments, the compositions of the present disclosure become associated with cells *in vivo* in a subject, for example, a human or mammal. In some embodiments, the compositions of the present disclosure become associated with cells *in vivo* in a subject by administering the compositions to the subject.

Various methods may be utilized to administer the compositions of the present disclosure to a subject. For instance, in some embodiments, the administration occurs by a method that includes, without limitation, oral administration, inhalation, subcutaneous administration, intravenous administration, intra-nasal administration, intra-dermal administration, trans-dermal administration, intraperitoneal administration, intramuscular administration, intrathecal injection, topical administration, central administration, peripheral administration, and combinations thereof.

In some embodiments, the compositions of the present disclosure can be associated with cells via delivery agents. In some embodiments, the delivery agents can include various types of particles and/or targeting agents associated with the compositions of the present disclosure. For instance, in some embodiments, the delivery agents can be particles associated with the compositions of the present disclosure.

The particles associated with the compositions of the present disclosure may have various sizes. For instance, in some embodiments, the particles associated with the compositions of the present disclosure are in the form of nanoparticles. In some embodiments, the nanoparticles have diameters ranging from about 50 nm to about 500 nm. In some embodiments, the nanoparticles have diameters of about 100 nm to about 150 nm. In some embodiments, the nanoparticles can be, for example, polymeric nanoparticles, lipid-based nanoparticles, single wall or multiwall carbon nanotubes, fullerenes, and combinations thereof.

Targeting agents may be associated with the compositions of the present disclosure in various manners. For instance, in some embodiments, the targeting agents associated with the compositions of the present disclosure may be on a surface of a particle. In some embodiments, the targeting agents associated with the compositions of the present disclosure may be covalently linked to the surface of the particle. In some embodiments, the targeting agents associated with the compositions of the present disclosure may be linked to the surface of the particle through a linker, such as polyethylene glycol (PEG).

In some embodiments, the compositions of the present disclosure include a solubilizing agent. Solubilizing agents generally refer to one or more compounds that are capable of facilitating the solubilization of the compositions of the present disclosure in liquid formulations. Solubilizing agents may also be referred to as co-solvents or carriers.

In some embodiments, the solubilizing agents of the present disclosure include water miscible organic solvents. In some embodiments, the solubilizing agents of the present disclosure include, without limitation, polyethylene glycol (e.g., PEG 400 and/or PEG 300), glycerin, propylene glycol, ethanol, sorbitol, polyoxyethylated glycerides (e.g., Labrafil M-2125CS), polyoxyethylated oleic glycerides (e.g., Labrafil M-1944CS, Polyoxyl 35 castor oil, and/or Cremophor EL), polysorbates (e.g., polysorbate 20 and/or polysorbate 80), sorbitan monooleate, hydroxypropyl-beta-cyclodextrin (HPCD), polyoxyl 40 hydrogenated castor oil (i.e., Cremophor RH 40), polyoxyl hydroxystearates (e.g., Solutol HS 15), and combinations thereof.

In some embodiments, the compositions of the present disclosure may be co-administered to a subject with additional materials, such as other active agents and/or inactive ingredients. In some embodiments, the administered compositions may be utilized to treat and/or prevent disorders in a subject. In some embodiments, the disorders include, without limitation, prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof. As such, in some embodiments, the present disclosure pertains to methods of treating or preventing one or more of the aforementioned disorders in a subject by administering the compositions of the present disclosure to the subject.

### Administration of Compositions to Subjects

The compositions of the present disclosure may be administered to a subject in order to treat or prevent various disorders in the subject. In some embodiments, the disorders can be, but are not limited to, prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof.

Various methods may be utilized to administer the compositions of the present disclosure to a subject. For instance, in some embodiments, the administration occurs by a method that includes, without limitation, oral administration, inhalation, subcutaneous administration, intravenous administration, intra-nasal administration, intra-dermal administration, trans-dermal administration, intraperitoneal administration, intramuscular administration, intrathecal injection, topical administration, central administration, peripheral administration, and combinations thereof. In some embodiments, the administering can be performed orally, intramuscularly, intranasally, subcutaneously, intra- or trans-dermally, intravenously, or combinations thereof.

In some embodiments, the compositions of the present disclosure can be administered to the subject via delivery agents. In some embodiments, the delivery agents can include various types of particles and/or targeting agents associated with the compositions of the present disclosure. For instance, in some embodiments, the delivery agents can be particles associated with the compositions of the present disclosure.

The particles associated with the compositions of the present disclosure may have various sizes. For instance, in some embodiments, the particles associated with the compositions of the present disclosure are in the form of nanoparticles. In some embodiments, the nanoparticles have diameters ranging from about 50 nm to about 500 nm. In some embodiments, the nanoparticles have diameters of about 100 nm to about 150 nm. In some embodiments, the nanoparticles can be, for example, polymeric nanoparticles, lipid-based nanoparticles, single wall or multiwall carbon nanotubes, fullerenes, and combinations thereof.

Targeting agents may be associated with the compositions of the present disclosure in various manners. For instance, in some embodiments, the targeting agents associated with the compositions of the present disclosure may be on a surface of a particle. In some embodiments, the targeting agents associated with the compositions of the present disclosure may be covalently linked to the surface of the particle. In some embodiments, the targeting agents associated with the compositions of the present disclosure may be linked to the surface of the particle through a linker, such as polyethylene glycol (PEG).

In some embodiments, the compositions of the present disclosure include a solubilizing agent. Solubilizing agents generally refer to one or more compounds that are capable of facilitating the solubilization of the compositions of the present disclosure in liquid formulations. Solubilizing agents may also be referred to as co-solvents or carriers.

In some embodiments, the solubilizing agents of the present disclosure include water miscible organic solvents. In some embodiments, the solubilizing agents of the present disclosure include, without limitation, polyethylene glycol (e.g., PEG 400 and/or PEG 300), glycerin, propylene glycol, ethanol, sorbitol, polyoxyethylated glycerides (e.g., Labrafil M-2125CS), polyoxyethylated oleic glycerides (e.g., Labrafil M-1944CS, Polyoxyl 35 castor oil, and/or Cremophor EL), polysorbates (e.g., polysorbate 20 and/or polysorbate 80), sorbitan monooleate, hydroxypropyl-beta-cyclodextrin (HPCD), polyoxyl 40 hydrogenated castor oil (i.e., Cremophor RH 40), polyoxyl hydroxystearates (e.g., Solutol HS 15), and combinations thereof.

### Applications and Advantages

The present disclosure can have various advantages. For instance, in some embodiments, the compositions of the present disclosure can decrease glucose release or deposition of fat from the liver (leading to a cleaner liver), increase the uptake of glucose in fat and muscle cells (which lowers blood sugar levels), and cause lower levels of insulin. Furthermore, in some embodiments, the compositions of the present disclosure can increase cellular glucose uptake independently of proximal insulin signaling pathways, through increasing the pAkt/Akt ratio. Moreover, since the compositions of the present disclosure are small molecule analogs of E4orf1, the compositions of the present disclosure do not have the same limitations as the E4orf1 protein (*e.g.*, having no receptor for the protein in human cells). As evident from the cellular glucose uptake and pAKT/AKT data in the Examples, the compositions of the present disclosure successfully enter cells and produce the aforementioned results.

Furthermore, in some embodiments, the compositions of the present disclosure can modulate glucose uptake levels. In some embodiments, the compositions of the present disclosure can increase cellular glucose uptake. In some embodiments, the aforementioned compositions increase cellular glucose uptake in fat cells, muscle cells, or liver cells. In some embodiments, the aforementioned compositions increase cellular glucose uptake independently of proximal insulin signaling pathways. In some embodiments, the aforementioned compositions mimic function of E4orf1. In some embodiments, the aforementioned compositions mimic function of E4orf1 in glucose uptake and insulin signaling in cells. In some embodiments, the aforementioned compositions cause an increase in pAkt/Akt in cells.

In some embodiments, the compositions of the present disclosure can reduce endogenous insulin levels in the blood. In some embodiments, the compositions of the present disclosure improve blood glucose levels by reducing high levels of glucose in the blood, or reducing the rise in glucose levels or the duration of glucose elevation expected after eating food. In some embodiments, the compositions of the present disclosure reduce glucose output from the liver. In some embodiments, the compositions of the present disclosure prevent an increase in blood insulin levels, reduce production and secretion of insulin while improving blood glucose levels, and thereby prevent damage to pancreatic cells that make insulin. In some embodiments, the compositions of the present disclosure reduce fat accumulation in the liver. In some embodiments, the compositions of the present disclosure prevent accumulation of fat in the liver or reduce accumulated lipid from the liver.

As such, the methods and compositions of the present disclosure can be utilized to treat or prevent various diseases and conditions, including, without limitation, prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof. More specifically, the methods and compositions of the present disclosure can be utilized as anti-diabetic agents to treat or prevent diabetes.

### Additional Embodiments

Reference will now be made to more specific embodiments of the present disclosure and experimental results that provide support for such embodiments. However, Applicants note that the disclosure below is for illustrative purposes only and is not intended to limit the scope of the claimed subject matter in any way.

### Example 1. Anti-Diabetic Property of Small Molecule Analogs of E4orf1 Protein

E4orf1 protein of human adenovirus 36 is necessary and sufficient to improve glycemic control through increased glucose uptake in adipose tissue and muscle cells and by reducing glucose output from the liver. E4orf1 bypasses the proximal insulin signaling pathway and uses distal insulin signaling pathway to uptake glucose. E4orf1 activates Ras which in turn activates PI3K to phosphorylate Akt and promotes the translocation of glucose transporter Glut4 to the membrane for glucose uptake. While many antidiabetic drugs are dependent on the proximal insulin signaling pathway, E4orf1 can uptake glucose independent of the proximal insulin signaling pathway. Hence, E4orf1 can be used as a potential drug to treat diabetes more effectively.

However, human cells lack receptors for the E4orf1 protein. Accordingly, Applicants developed chemical analogs of E4orf1, termed BT324, BT325, BT326, BT327, BT328, and BT329. These analogs were tested *in vitro* to check if they mimic the function/action of E4orf1 on glucose uptake and insulin signaling.

As disclosed herein, of the six E4orf1 analogs tested, four analogs, BT324, BT325, BT327, and BT329 significantly increase glucose uptake repeatedly. Described and illustrated in further detail herein are the six analogs and the results from testing them on 3T3L1 preadipocytes with DMSO and insulin used as controls.

### Example 1.1. Methods

The following E4orf1 analogs, as shown in **FIG.** 1 and described below in Table 1, were developed and synthesized.

**Table 1**

| **Compounds** | **Molecular Weight** |
|---|---|
| BT324 | 238.62 |
| BT325 | 224.60 |
| BT326 | 237.64 |
| BT327 | 223.61 |
| BT328 | 245.08 |
| BT329 | 160.18 |

### Example 1.2. Dissolving Analogs in DMSO to Treat Cells

All analogs were dissolved in 2 ml of DMSO in dark (to prevent exposure to light). They were aliquoted (200 µl) to avoid repeated freeze and thaw and stored at 4 °C.

### Example 1.3. Experiments - Glucose Uptake and Western Blot

3T3L1 preadipocytes (passage number 8 below) were used to treat with analogs. As control, cells were treated with equal volumes of DMSO. As positive control, cells were treated with 100 nM insulin. Cells at 70% confluency were treated with analogs (2 µl/ml of media) in dark for 24 hr. Post 24 hr treatment, cells were used either to determine glucose uptake or to obtain cell lysates for determining pAKT abundance by Western blot analysis.

### Example 1.4. Results

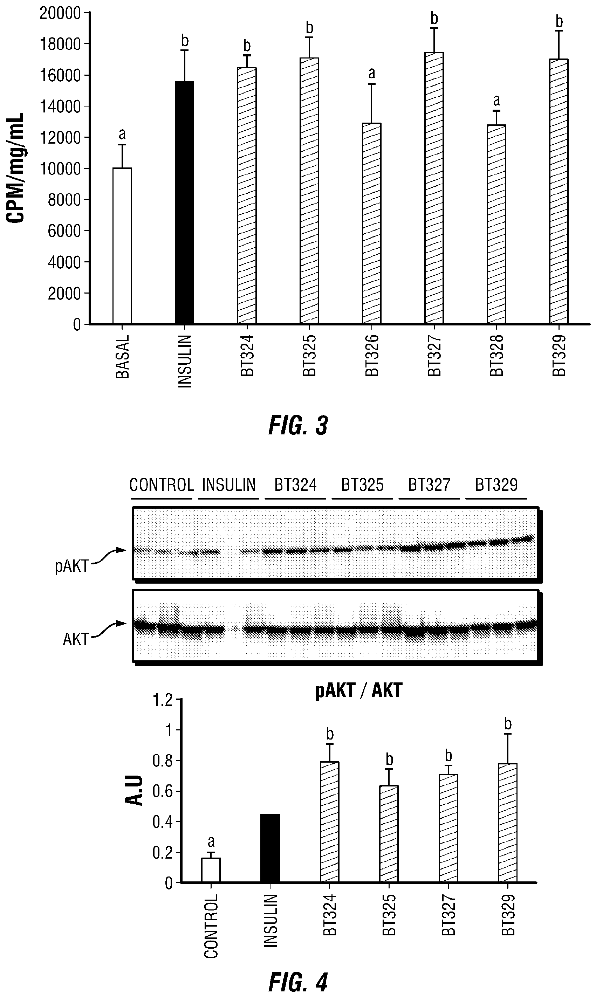

Of the six E4orf1 analogs tested, four analogs, BT324, BT325, BT327 and BT329 significantly increased glucose uptake repeatedly (individual experiments repeated 3 times), as shown in **FIG. 3**. Cell signaling Western blot data showed significant increase in pAkt/Akt for the four analogs, as shown in **FIG. 4**.

### Example 1.5. Conclusion

E4orf1 analogs BT324, BT325, BT327, and BT329 increase cellular glucose uptake and enhance insulin signaling protein, similar to that by E4orf1. These analogs are good candidates as anti-diabetic agents to treat or prevent diabetes.

### Example 2. Small Molecule Candidates

This Example describes developing small molecule candidates that mimic the action of E4orf1.

### Example 2.1. Development of Small Molecules

While E4orf1 can improve glycemic control, delivering this exogenous protein to cells is challenging. To address this challenge, Applicants considered a peptidomimetic approach, which has shown to improve safety and minimize proteolytic susceptibility. Applicants successfully addressed this challenge by developing small molecule candidates that mimic the action of E4orf1. This will provide information to take the lead molecules towards clinical studies.

The exact crystal structure of the E4orf1 is not known. Hence, Applicants employed the DNA and amino acid sequence of E4orf1 protein to construct homology modeling using Applicants' newly developed software. In this process, Applicants took base pairs of DNA and used European Bioinformatics Institute (EMBL-EBI) database search for the types of amino acid sequence that could be aligned with the existing proteins. Ad36 E4orf1 Protein translation sequence is:

MAESLYAFIDSPGGIAPVQEGASNRYIFFCPESFHIPPHGVILLHLRVSVLVPTGYQGRFM
ALNDYHARGILTQSDVIFAGRRHDLSVLLFNHTDRFLYVREGHPVGTLLLERVIFPSVRI
ATLV (SEQ ID NO: 1)

After determining the protein sequence, Applicants refined using Molsoft ICM and Molecular Operating Environment and remodeled the active site and generated the active site of the protein in silico as previously described. Next, Applicants screened their small libraries of chemical compounds, which are theoretical molecules. After identifying the hits in-sillico, Applicants synthesized the molecules based on new reactions developed by Applicants.

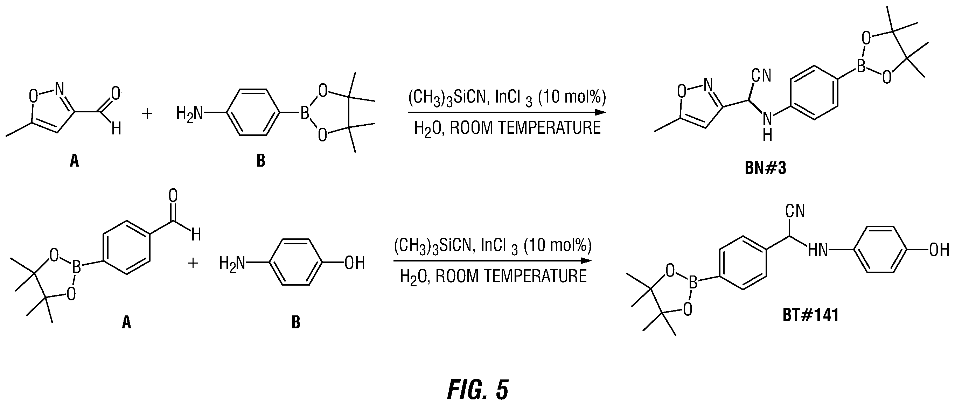

Amino nitriles based pharmacophore groups are well accepted in drug discovery. All compounds Applicant synthesized contain alpha-aminonitrile pharmacophore groups and all derivatives are substituted with amino groups containing boronic acids (**FIG. 5**). Hence, the alpha-aminonitrile containing boronic acid derivatives are new compounds. These molecules were characterized using routine analytical tools used in medicinal chemistry, such as Nuclear Magnetic Resonance (NMR), both 1H proton and 13Carbon NMR, and High Resolution Mass Spectroscopy (HRMS). The purity of the compounds was determined by High Performance Liquid Chromatography (HPLC).

The compounds were found to be stable, white solid, not pyrophoric, and not water reactive. The compounds were also found to be water soluble, and with a log P value under the therapeutic index (3.5-4.5).

Applicants' lab has the capacity to develop clinical grade compounds needed (BN#3 and BT#141). The general procedure to synthesize these amino nitriles is as follows (**FIG. 5**). To a 10 mL round bottomed flask containing aldehyde A (0.5 mmol, 1.0 eq), amine B (0.5 mmol, 1.0 eq), Trimethylsilyl cyanide (TMSCN; 0.6 mmol, 1.2 eq) and water (2 mL), InCl₃ (Indium Chloride; 0.05 mmol, 10 mol%) is added as the catalyst. The resulting mixture is stirred overnight at room temperature. After the reaction is complete, the crude solid product is filtered and washed by water and hexane, and further purified by silica gel chromatography using ethylacetate and hexanes as solvent. All compounds are characterized by using proton, Carbon NMR and HRMS.

### Example 2.2. In Vitro Screening of Small Molecules

To determine optimal dose and toxicity, 3T3-L1 pre-adipocytes were exposed to different concentrations (5 µM, 10 µM, 20 µM, 50 µM and 100 µM) of 5 different small molecules in dimethyl sulfoxide (DMSO). Control cells were exposed to DMSO alone. Concentrations of 50 µM and 100 µM showed extreme toxicity and cell death after day 2 of exposure, while exposure to other concentrations was followed until day 5 and based on percent cell survival, 20 µM concentration was determined to be optimal for testing glucose disposal with these compounds.

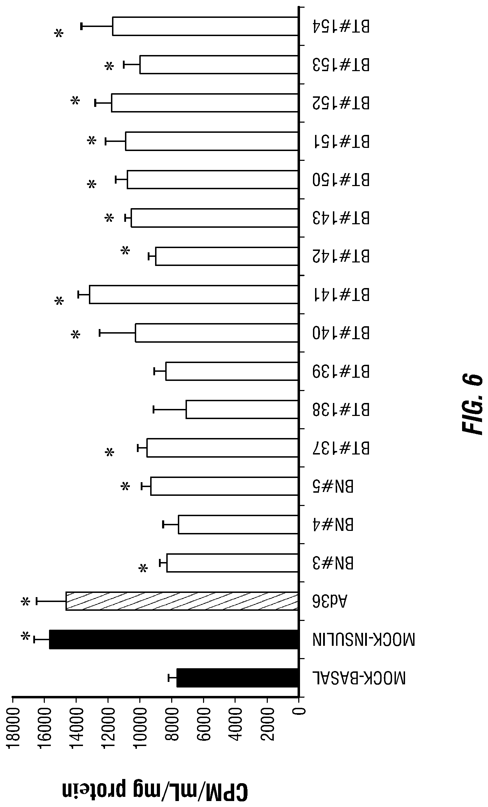

Next, over time, Applicants tested cellular glucose uptake with 41 different E4orf1 analog small molecules in 3T3-L1 pre-adipocytes, mature adipocytes and murine skeletal muscle cells. Cells were exposed to 20 µM/well of E4orf1 analogs. Glucose uptake by cells was determined 24h later. Insulin and human adenovirus Ad36 were used as positive controls for glucose uptake. Data from a batch of 15 compounds screened are presented in **FIG. 6**. The * indicates statistical significance after Bonferroni correction for multiple comparisons. Data on cell signaling including Ras, pAKT, Glut4 for selected compounds are not presented due to space constrains, as this PAR does not focus on mechanism of action.

Applicants conducted glucose uptake assays for compounds several separate times. BN#3 and BT#141 had about 70% greater glucose uptake compared to control in other replications not presented herein.

### Example 2.3. Candidate and Lead Molecule Identification

Of the 41 different small molecules tested, Applicants selected 12 candidate compounds, 4 aminonitrile (BN#3, BN#5, BT#141, BT#143) and 8 chromene (BT#156, BT#158, BT#166, BT#170, BT#181, BT#185, BT#186 and BT#188) that displayed consistently significant increase in cellular glucose when tested 3 or more times in different cell types. From the 12 candidate compounds Applicants tested 4 molecules (BN#3, BT#141, BT#143 and BT#181) *in vivo*.

### Example 2.4. Improvement of Glucose Disposal by Lead Molecules

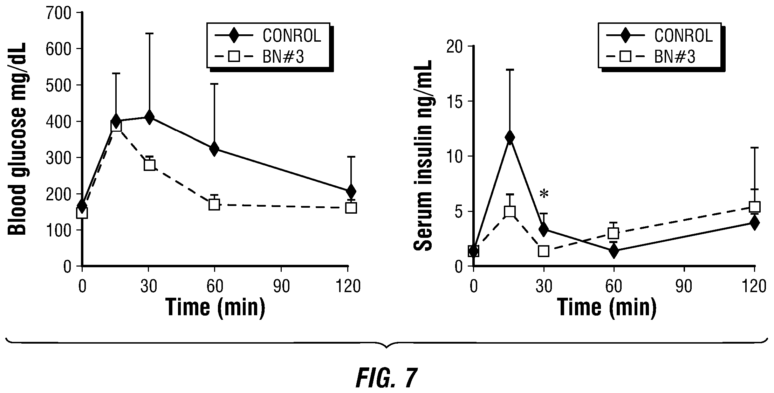

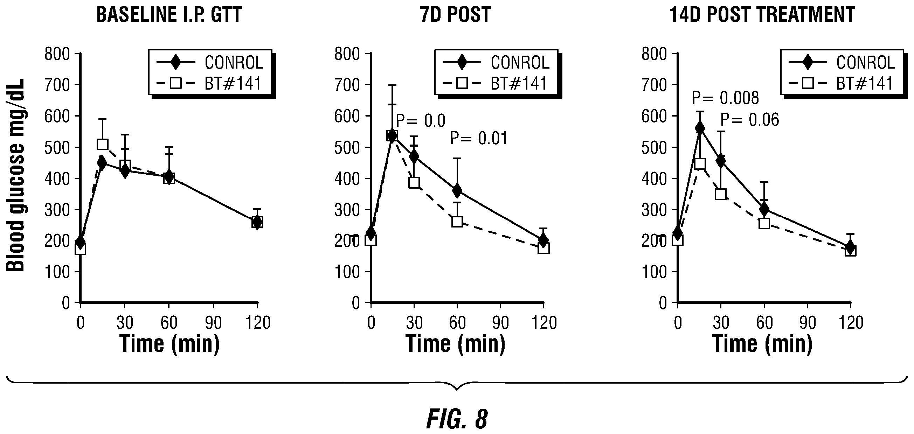

To determine improvement in glucose disposal in *vivo*, 10 wk old C57BL/6J male mice (Jax Laboratories) on a 60% kcal high-fat diet (Research diets, Inc.) since 6 weeks of age were divided into weight-matched groups. In individual experiments, mice were treated with daily injection of 4 mg/kg of respective small molecules (BN#3, BT#141, BT#143 and BT#181) or DMSO alone as control. Improvement in glycemic control was determined by glucose tolerance test (GTT) performed at baseline (prior to treatment), 7 days and/or 14 days post-treatment. Insulin-independent action of the small molecules was also measured by collecting blood during GTT and quantifying serum insulin by enzyme-linked immunosorbent assay (ELISA). Candidate compounds BT#143 and BT#181 did not show any significant improvement in glucose clearance compared to control (DMSO) mice (data not shown). However, BN#3 and BT#141 showed improved glycemic control compared with control mice, respectively (**FIG. 7** and **FIG. 8**).

Without further elaboration, it is believed that one skilled in the art can, using the description herein, utilize the present disclosure to its fullest extent. The embodiments described herein are to be construed as illustrative and not as constraining the remainder of the disclosure in any way whatsoever. While the embodiments have been shown and described, many variations and modifications thereof can be made by one skilled in the art without departing from the spirit and teachings of the invention. Accordingly, the scope of protection is not limited by the description set out above, but is only limited by the claims, including all equivalents of the subject matter of the claims. The disclosures of all patents, patent applications and publications cited herein are hereby incorporated herein by reference, to the extent that they provide procedural or other details consistent with and supplementary to those set forth herein.

### Clauses

1. A method of modulating cellular glucose uptake, wherein the method comprises:
   associating cells with a composition, wherein the composition comprises a compound selected from the group consisting of: 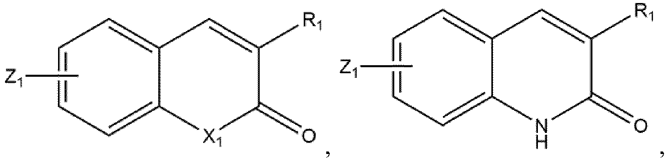 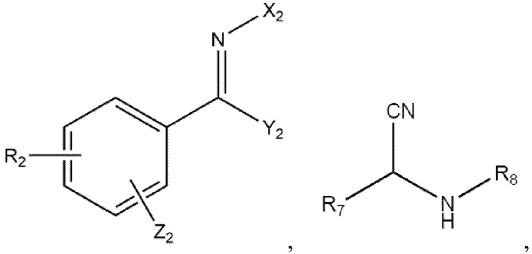 or combinations thereof,
   wherein R₁ is selected from the group consisting of an alkyl group, an alkene group, an alkyne group, an alkoxyl group, an aldehyde, a carboxyl group, COOR₃, COOH, hydrogen, SH, SH derivatives, SR₆, or OH,
   wherein R₃ is selected from the group consisting of an alkyl group, an alkene group, an alkyne group, a methyl group, an ethyl group, a propyl group, a butyl group, or a pentyl group,
   wherein R₆ is selected from the group consisting of an alkyl group, an alkene group, an alkyne group, an alkoxyl group, an aldehyde, a carboxyl group, COOR₃, COOH, hydrogen, or OH,
   wherein Z₁ and Z₂ are each independently selected from the group consisting of a halogen, hydrogen, SH, SH derivatives, an alkyl group, an alkene group, an alkyne group, or SR₆,
   wherein X₁ is selected from the group consisting of O, NH, NR₄, SH, SH derivatives, CH₂, SR₆, or S, wherein R₄ is selected from the group consisting of an alkyl group, an alkene group, an alkyne group, or a hydroxyl group,
   wherein R₂ is selected from the group consisting of OR₅, an alkyl group, an alkene group, an ethynyl group, an alkyne group, SH, SH derivatives, SR₆, or COOR₅,
   wherein R₅ is selected from the group consisting of hydrogen, an alkyl group, an alkene group, an alkyne group, a methyl group, an ethyl group, a propyl group, a butyl group, and a pentyl group,
   wherein X₂ and Y₂ are each independently selected from the group consisting of OH, NH₂, NR₄, an alkyl group, an alkene group, an alkyne group, a methyl group, an ethyl group, a propyl group, a butyl group, a pentyl group, SH, SH derivatives, or SR₆, and
   wherein R₇ and R₈ are each independently selected from the group consisting of 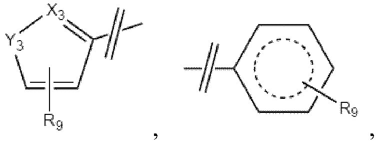 an alkyl group, an alkene group, an alkyne group, an alkoxyl group, an ethynyl group, an aldehyde, a carboxyl group, OH, COOR₃, COOH, hydrogen, SH, SH derivatives, SR₆, OR₅, or COOR₅,
   wherein X₃ is selected from the group consisting of N, CH, SH, SH derivatives, or P,
   wherein Y₃ is selected from the group consisting of O, NH, NR₄, SH, SH derivatives, SR₆, CH₂, or S, and
   wherein R₉ is selected from the group consisting of an alkyl group, an alkene group, an alkyne group, an alkoxyl group, an ethynyl group, an aldehyde, a carboxyl group, COOR₃, COOH, hydrogen, SH, SH derivatives, SR₆, OR₅, COOR₅, CH₃, OH, or 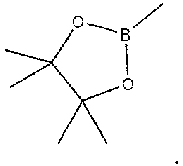
2. The method of clause 1, wherein the composition comprises a compound selected from the group consisting of: 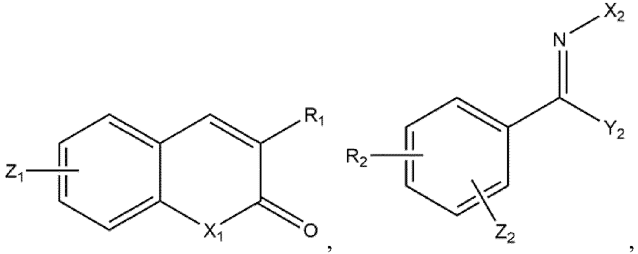 or combinations thereof.
3. The method of clause 2, wherein R₁ is selected from the group consisting of COOCH₃ or COOH,
   wherein R₂ is selected from the group consisting of OCH₃ or ethynyl (CCH),
   wherein X₁ is selected from the group consisting of O or NH,
   wherein X₂ is OH,
   wherein Z₁ is Cl,
   wherein Z₂ is selected from the group consisting of Br or hydrogen, and
   wherein Y₂ is NH₂.
4. The method of clause 1, wherein R₇ is selected from the group consisting of: 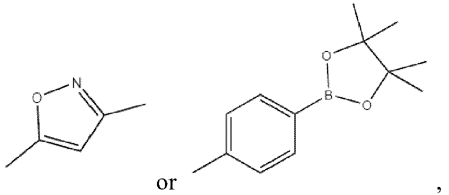 and
   wherein R₈ is selected from the group consisting of: 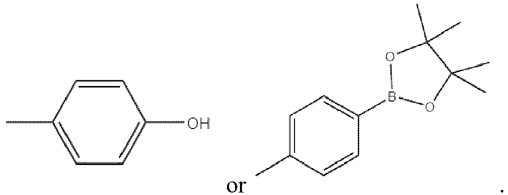
5. The method of clause 1, wherein the composition comprises a compound selected from the group consisting of 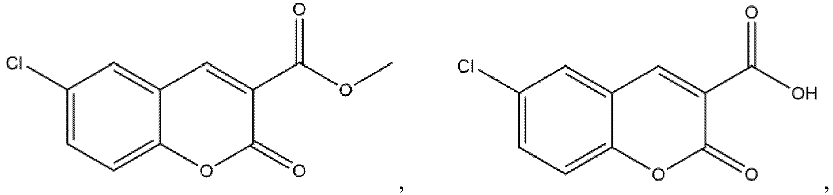 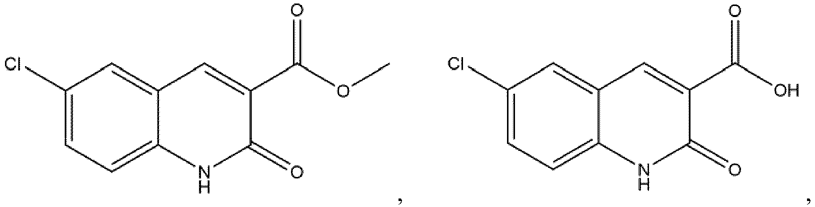 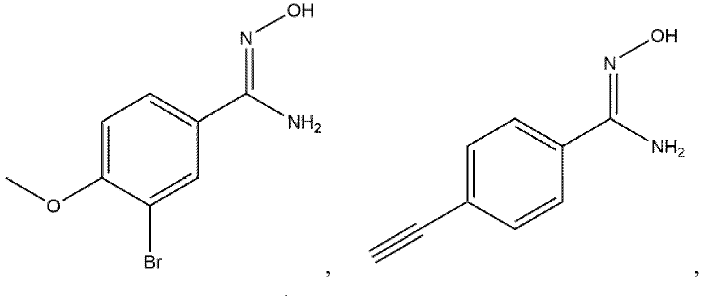 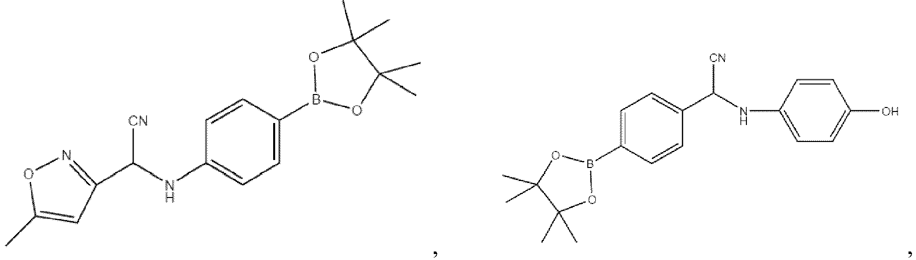 or combinations thereof.
6. The method of clause 1, wherein the composition increases cellular glucose uptake.
7. The method of clause 6, wherein the composition increases cellular glucose uptake independently of proximal insulin signaling pathways.
8. The method of clause 1, wherein the composition mimics function of early 4 open reading frame 1 protein of human adenovirus 36 (E4orf1).
9. The method of clause 1, wherein the composition causes an increase in pAkt/Akt ratios.
10. The method of clause 1, wherein the cells are fat cells, muscle cells, or liver cells, and wherein the composition causes an increase in cellular glucose uptake in the fat cells, muscle cells, or liver cells.
11. The method of clause 1, wherein the associating is performed *in vitro*.
12. The method of clause 1, wherein the associating is performed *in vivo* in a subject.
13. The method of clause 12, wherein the associating comprises administering the composition to the subject.
14. The method of clause 13, wherein the administering is performed orally, intramuscularly, intranasally, subcutaneously, intra- or trans-dermally, intravenously, or combinations thereof.
15. The method of clause 12, wherein the composition is used to treat or prevent a disorder in the subject.
16. The method of clause 15, wherein the disorder is prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof.
17. The method of clause 12, wherein the composition reduces endogenous insulin levels in the blood.
18. The method of clause 12, wherein the composition improves blood glucose levels by reducing high levels of glucose in the blood, or reducing the rise in glucose levels or the duration of glucose elevation expected after eating food, or reducing glucose output from the liver.
19. The method of clause 12, wherein the composition prevents an increase in blood insulin levels.
20. The method of clause 12, wherein the composition reduces production and secretion of insulin while improving blood glucose levels, thereby preventing damage to pancreatic cells that make insulin.
21. The method of clause 12, wherein the composition reduces fat accumulation in the liver or reduces accumulated lipid from the liver.
22. The method of clause 12, wherein the composition prevents accumulation of fat in the liver.
23. The method of clause 1, wherein the composition is associated with a delivery agent.
24. The method of clause 23, wherein the delivery agent is a nanoparticle.
25. The method of clause 1, wherein the composition comprises at least one solubilizing agent selected from the group consisting of polyethylene glycol, glycerin, propylene glycol, ethanol, sorbitol, polyoxyethylated glycerides, polyoxyethylated oleic glycerides, polysorbates, sorbitan monooleate, hydroxypropyl-beta-cyclodextrin (HPCD), polyoxyl 40 hydrogenated castor oil, polyoxyl hydroxystearates, or combinations thereof.
26. A method of treating or preventing a disorder in a subject, wherein the method comprises:
   administering a composition to the subject, wherein the composition comprises a compound selected from the group consisting of: 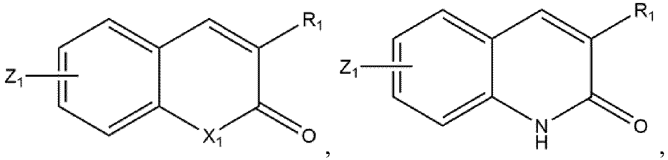 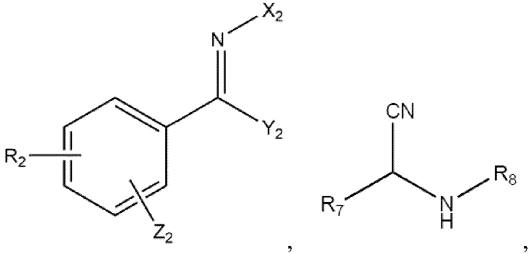 or combinations thereof,
   wherein R₁ is selected from the group consisting of an alkyl group, an alkene group, an alkyne group, an alkoxyl group, an aldehyde, a carboxyl group, COOR₃, COOH, hydrogen, SH, SH derivatives, SR₆, or OH,
   wherein R₃ is selected from the group consisting of an alkyl group, an alkene group, an alkyne group, a methyl group, an ethyl group, a propyl group, a butyl group, or a pentyl group,
   wherein R₆ is selected from the group consisting of an alkyl group, an alkene group, an alkyne group, an alkoxyl group, an aldehyde, a carboxyl group, COOR₃, COOH, hydrogen, or OH,
   wherein Z₁ and Z₂ are each independently selected from the group consisting of a halogen, hydrogen, SH, SH derivatives, an alkyl group, an alkene group, an alkyne group, or SR₆,
   wherein X₁ is selected from the group consisting of O, NH, NR₄, SH, SH derivatives, CH₂, SR₆, or S, wherein R₄ is selected from the group consisting of an alkyl group, an alkene group, an alkyne group, or a hydroxyl group,
   wherein R₂ is selected from the group consisting of OR₅, an alkyl group, an alkene group, an ethynyl group, an alkyne group, SH, SH derivatives, SR₆, or COOR₅,
   wherein R₅ is selected from the group consisting of hydrogen, an alkyl group, an alkene group, an alkyne group, a methyl group, an ethyl group, a propyl group, a butyl group, and a pentyl group,
   wherein X₂ and Y₂ are each independently selected from the group consisting of OH, NH₂, NR₄, an alkyl group, an alkene group, an alkyne group, a methyl group, an ethyl group, a propyl group, a butyl group, a pentyl group, SH, SH derivatives, or SR₆, and
   wherein R₇ and R₈ are each independently selected from the group consisting of 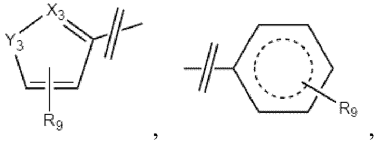 an alkyl group, an alkene group, an alkyne group, an alkoxyl group, an ethynyl group, an aldehyde, a carboxyl group, OH, COOR₃, COOH, hydrogen, SH, SH derivatives, SR₆, OR₅, or COOR₅,
   wherein X₃ is selected from the group consisting of N, CH, SH, SH derivatives, or P,
   wherein Y₃ is selected from the group consisting of O, NH, NR₄, SH, SH derivatives, SR₆, CH₂, or S, and
   wherein R₉ is selected from the group consisting of an alkyl group, an alkene group, an alkyne group, an alkoxyl group, an ethynyl group, an aldehyde, a carboxyl group, COOR₃, COOH, hydrogen, SH, SH derivatives, SR₆, OR₅, COOR₅, CH₃, OH, or 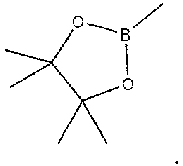
27. The method of clause 26, wherein the disorder is prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction characterized by fatty liver and/or insulin resistance, polycystic ovary syndrome, or combinations thereof.
28. The method of clause 26, wherein the administering is performed orally, intramuscularly, intranasally, subcutaneously, intra- or trans-dermally, intravenously, or combinations thereof.
29. The method of clause 26, wherein the composition comprises a compound selected from the group consisting of: 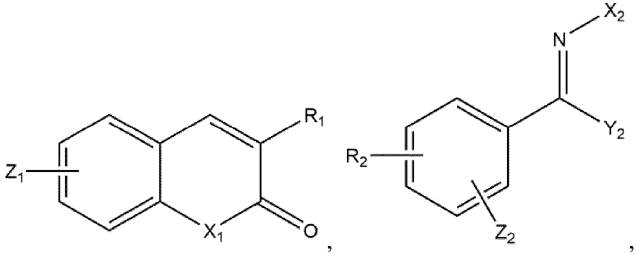 or combinations thereof.
30. The method of clause 29, wherein R₁ is selected from the group consisting of COOCH₃ or COOH,
   wherein R₂ is selected from the group consisting of OCH₃ or ethynyl (CCH),
   wherein X₁ is selected from the group consisting of O or NH,
   wherein X₂ is OH,
   wherein Z₁ is Cl,
   wherein Z₂ is selected from the group consisting of Br or hydrogen, and
   wherein Y₂ is NH₂.
31. The method of clause 26, wherein R₇ is selected from the group consisting of: 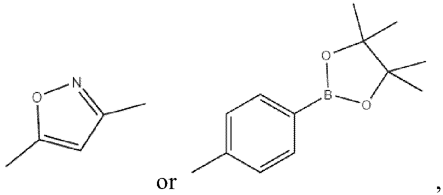 and
   wherein R₈ is selected from the group consisting of: 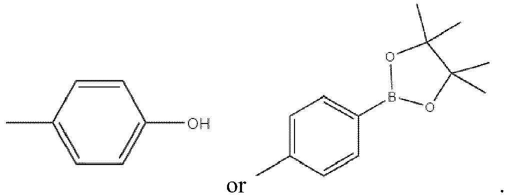
32. The method of clause 26, wherein the composition comprises a compound selected from the group consisting of 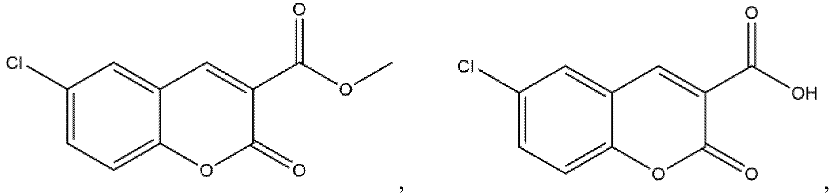 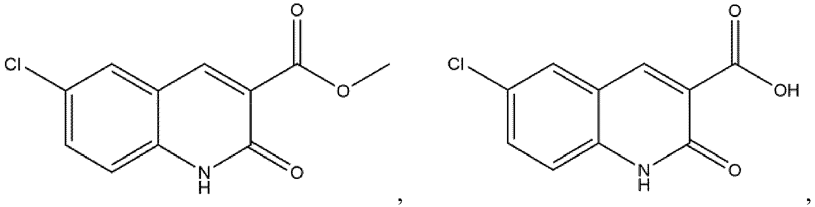 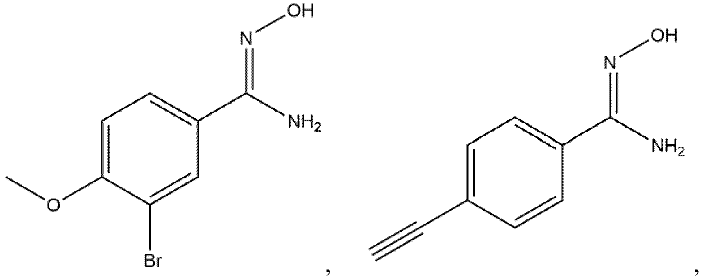 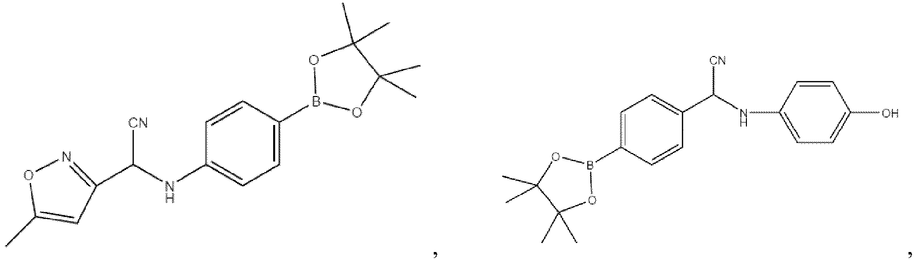 or combinations thereof.
33. The method of clause 26, wherein the composition mimics function of early 4 open reading frame 1 protein of human adenovirus 36 (E4orf1).
34. The method of clause 26, wherein the composition causes an increase in pAkt/Akt ratios.
35. The method of clause 26, wherein the composition reduces endogenous insulin levels in the blood.
36. The method of clause 26, wherein the composition improves blood glucose levels by reducing high levels of glucose in the blood, or reducing the rise in glucose levels or the duration of glucose elevation expected after eating food, or reducing glucose output from the liver.
37. The method of clause 26, wherein the composition prevents an increase in blood insulin levels.
38. The method of clause 26, wherein the composition reduces production and secretion of insulin while improving blood glucose levels, thereby preventing damage to pancreatic cells that make insulin.
39. The method of clause 26, wherein the composition reduces fat accumulation in the liver or reduce accumulated lipid from the liver.
40. The method of clause 26, wherein the composition prevents accumulation of fat in the liver.
41. The method of clause 26, wherein the composition is associated with a delivery agent.
42. The method of clause 41, wherein the delivery agent is a nanoparticle.
43. The method of clause 26, wherein the composition comprises at least one solubilizing agent selected from the group consisting of polyethylene glycol, glycerin, propylene glycol, ethanol, sorbitol, polyoxyethylated glycerides, polyoxyethylated oleic glycerides, polysorbates, sorbitan monooleate, hydroxypropyl-beta-cyclodextrin (HPCD), polyoxyl 40 hydrogenated castor oil, polyoxyl hydroxystearates, or combinations thereof.
44. The method of clause 26, wherein the composition modulates cellular glucose uptake levels.
45. The method of clause 26, wherein the composition increases cellular glucose uptake levels.
46. The method of clause 26, where the composition increases cellular glucose uptake independently of proximal insulin signaling pathways.

## Claims

1. A composition for use in treating or preventing a disorder in a subject, wherein the composition comprises a compound selected from the group consisting of 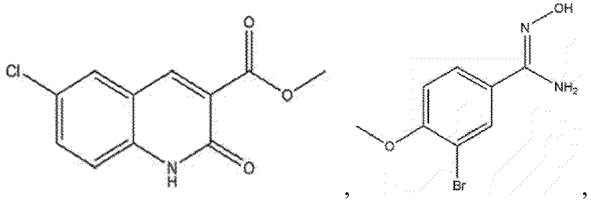 and 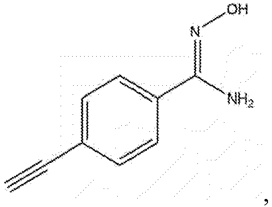 or combinations thereof, and wherein the disorder is prediabetes, diabetes type 1, diabetes type 2, insulin resistance, hyperinsulinemia, hyperglycemia, metabolic syndrome, hepatic steatosis, non-alcoholic fatty liver disease (NAFLD), non-alcoholic steato-hepatitis (NASH), liver dysfunction **characterized by** fatty liver and/or insulin resistance, polycystic ovary syndrome, or combinations thereof.

2. The composition for use of claim 1, wherein the composition is for administration orally, intramuscularly, intranasally, subcutaneously, intra- or trans-dermally, intravenously, or combinations thereof.

3. The composition for use of claim 1 or 2, wherein the compound is:

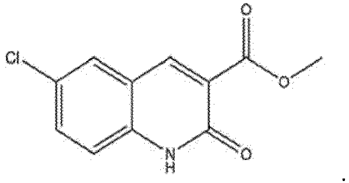

4. The composition for use of claim 1 or 2, wherein the compound is:

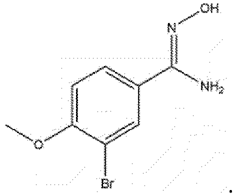

5. The composition for use of claim 1 or 2, wherein the compound is:

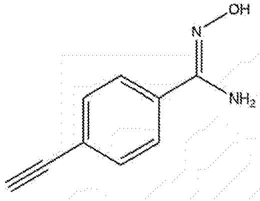

6. A method of modulating cellular glucose uptake *in vitro,* wherein the method comprises: associating cells with a composition, wherein the composition comprises a compound selected from the group consisting of: 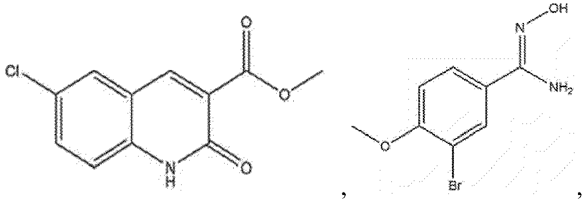 and 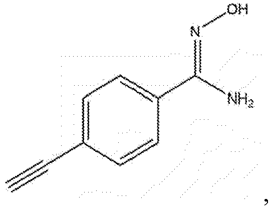 or combinations thereof.

7. The method of claim 6, wherein the cells are fat cells, muscle cells or liver cells.

8. The method of claim 6 or 7, wherein the compound is:

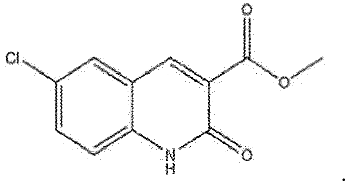

9. The method of claim 6 or 7, wherein the compound is:

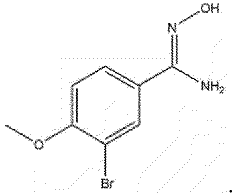

10. The method of claim 6 or 7, wherein the compound is:
